(19) Europäisches Patentamt
European Patent Office
Office européen des brevets

(11) **EP 1 161 202 B1**

(12) **EUROPÄISCHE PATENTSCHRIFT**

(45) Veröffentlichungstag und Bekanntmachung des Hinweises auf die Patenterteilung:
**15.08.2007 Patentblatt 2007/33**

(21) Anmeldenummer: **00910858.0**

(22) Anmeldetag: **17.03.2000**

(51) Int Cl.:
*A61F 2/04* *(2006.01)*    *A61B 5/20* *(2006.01)*

(86) Internationale Anmeldenummer:
**PCT/EP2000/002407**

(87) Internationale Veröffentlichungsnummer:
**WO 2000/056246 (28.09.2000 Gazette 2000/39)**

(54) **KÜNSTLICHES HARNABLEITUNGSSYSTEM**

ARTIFICIAL URINARY DIVERSION SYSTEM

SYSTEME ARTIFICIEL DE DERIVATION URINAIRE

(84) Benannte Vertragsstaaten:
**AT BE CH CY DE DK ES FI FR GB GR IE IT LI LU MC NL PT SE**

(30) Priorität: **18.03.1999 DE 19912218**
**19.03.1999 DE 19912472**

(43) Veröffentlichungstag der Anmeldung:
**12.12.2001 Patentblatt 2001/50**

(73) Patentinhaber:
• **Wassermann, Helmut**
**81739 München (DE)**
• **Jocham, Dieter**
**23568 Lübeck (DE)**

(72) Erfinder:
• **Wassermann, Helmut**
**81739 München (DE)**
• **Jocham, Dieter**
**23568 Lübeck (DE)**

(74) Vertreter: **Kunz, Herbert**
**HAMMONDS**
**Karl-Scharnagl-Ring 7**
**80539 München (DE)**

(56) Entgegenhaltungen:
WO-A-95/11637        WO-A-98/35633
DE-A- 3 526 164      DE-A- 3 932 718
US-A- 4 311 659      US-A- 5 813 410

EP 1 161 202 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein künstliches Harnableitungssystem.

Bei Patienten mit Erkrankungen der Harnblase gibt es eine große Anzahl von Befunden, die eine Entfernung der eigenen Blase erforderlich machen. In dieser Situation ist eine Harnableitung unter Herstellung verschiedener Formen von Reservoirs notwendig. Unterschieden werden sogenannte nasse Ableitungen mit direkter Ableitung des Urins über die Harnleiter, die in die Bauchwand eingepflanzt sind oder unter Zwischenschaltung eines ausgeschalteten Darmstücks, in das die Harnleiter eingepflanzt werden und das seinerseits in die Bauchwand eingepflanzt wird.

In beiden Fällen wird der Urin in einem auf die Mündungsstelle aufgeklebten Urinbeutel gesammelt.

Alternativ werden die Harnleiter in den Enddarm eingepflanzt oder -in den letzten Jahren mit zunehmender Verwendung- in Ersatzblasen, die aus ausgeschalteten Darmstücken gebildet werden.

Diese Ersatzblasen werden entweder an die körpereigene Harnröhre angeschlossen oder unter Bildung eines geeigneten selbsthaltenden Verschlußmechanismus an der Bauchhaut z.B. in Bereich des Nabels ausgeleitet.

In der US 5,813,410 ist eine mechanische Steuerung zum Betreiben einer künstlichen Harnableitungseinrichtung beschrieben. Die hierfür notwendige Steuerung ist sehr aufwendig und kann keinen anatomischen Gegebenheiten Rechnung tragen.

In der WO 98/35633 ist ein künstliches Harnableitungssystem beschrieben, welches eine künstliche Harnblase aufweist. Hierbei besteht die Harnableitungseinrichtung aus einem Mantelsystem, in dem die Harnblase integriert ist. Sowohl die Mantelfläche als auch die Harnblase sind flexibel ausgestaltet, wodurch allerdings die Fixierung im menschlichen Körper problematisch ist.

Typische Indikationen für einen Ersatz der körpereigenen Harnblase stellen fortgeschrittene Tumorerkrankungen der Harnblase dar, daneben gibt es aber auch Mißbildungen, entzündlich bedingte Schädigungen der Harnblase und funktionelle Störungen, wie z.B. Blasenentleerungsstörungen und Schrumpfblasenbildungen bei querschnittsgelähmten Patienten.

Es ist somit Aufgabe der vorliegenden Erfindung, ein künstliches Harnableitungssystem zu schaffen, welches an unterschiedlichen Formgebungen unterschiedlicher Personen anpaßbar ist und ein größtmögliches Befüllungsvolumen aufweisen kann.

Ferner ist es eine Aufgabe der vorliegenden Erfindung, daß künstliche Harnableitungssysteme derart anpaßbar gestaltet werden können, ohne vorherige direkte oder indirekte Bestimmung des evtl. zur Verfügung stehenden Volumens für das künstliche Harnableitungssystems, daß während der Operationsphase erst das in der betreffenden Person zur Verfügung stehende Volumen bestmöglich ermittelt und ausgenutzt wird.

Gelöst werden diese Aufgaben mit den Merkmalen des Anspruchs 1.

Anmeldungsgemäß weist der zwischen dem ersten und dem dritten Bereich angeordnete zweite Bereich eine minimale Querschnittsfläche $Q_2$ auf, welche kleiner als die maximale Querschnittsfläche $Q_3$ des dritten Bereichs ist. Dadurch wird erreicht, daß eine Form vorgegeben wird, welche beinahe auf jeden Menschen angepaßt werden kann und insbesondere wird dadurch erreicht, daß ein größtmögliches Befüllungsvolumen bereitgestellt werden kann, und zwar bei gleichzeitiger Beachtung medizinischer Vorgaben, wie beispielsweise, der nach erfolgter Operation seitlich an dem zweiten Bereich vorbeilaufenden Arterien und Darm, auf die kein Druck ausgeübt werden darf. Hierzu ist zu beachten, daß der dritte Bereich beim aufrecht stehenden Menschen oberhalb des zweiten und ersten Bereichs angeordnet wird. Auch wird beispielsweise für den Fall, bei dem evtl. der erste Bereich eine maximale Querschnittsfläche $Q_1$ als der zweite Bereich aufweist, erreicht, daß eine sogenannte Einschnürung im zweiten Bereich, welche für die vorbeilaufenden Arterien und/oder Darm und Nieren notwendig ist, bereitgestellt wird, und mit dem ersten Bereich eine Lagefixierung beispielsweise am Schambein (Symphysis Pubica) möglich ist.

Weitere vorteilhafte Ausgestaltungen der folgenden Erfindung sind Gegenstand der Unteransprüche.

Wird gemäß Anspruch 2 der erste, der zweite und der dritte Bereich modular zusammengesetzt bzw. modular zusammensetzbar ausgestaltet, und darauf geachtet, daß' die jeweiligen Übergangsflächen zwischen den einzelnen Bereichen derart aufeinander abgestimmt sind, daß ein stetiger Übergang erfolgt, so wird der Vorteil erzielt, daß je nach räumlichen Vorgaben des Patienten individuell die einzelnen Bereiche der Harnableitungseinrichtung zusammengestellt werden können und somit wiederum, optimal den anatomischen Vorgaben der zu behandelnden Person Rechnung getragen werden kann.

Wird gemäß Anspruch 4 eine Fluidführung vorgesehen, welche sich von der Harnblase bis zum Ausgang im ersten Bereich erstreckt, so wird der natürlichen Anatomie weitestgehend entsprochen, daß heißt, der bei einer aufrecht stehenden Person unterste erste Bereich kann dann direkt mit der vorhandenen Harnröhre verbunden werden, ohne zusätzliche Verbindungselemente zwischen der Harnröhre und dem Ausgang im ersten Bereich verwenden zu müssen, was evtl. weitere medizinische Komplikationen mit sich bringen könnte.

Wird gemäß Anspruch 5 in dem dritten Bereich ein Aktor oder eine Pumpe vorgesehen, so muß zum einen keine externe Pumpe bereitgestellt werden und der erste und der zweite Bereich wird hinsichtlich der Formgebung nicht negativ beeinflußt. Außerdem ist mit der vorteilhaften Ausgestaltung, daß ein Aktor oder eine Pumpe im dritten Bereich vorgesehen ist, dem Umstand Rechnung getragen, daß dieser größtmöglich ausgestaltete, dritte Bereich am ehesten genügend Platz für die Aufnahme einer Pumpe hat, ohne dabei unverhältnismäßig großen bzw. negativen Einfluß auf die Formgebung nehmen zu müssen.

Wird gemäß Anspruch 6 die Pumpe als Teleskopvorrichtung ausgebildet, so wird der Vorteil erreicht, daß nahezu das gesamte Volumen des dritten Bereichs zur Befüllung der darin enthaltenen Harnblase herangezogen werden kann. Laborversuche haben bereits gezeigt, daß mit einer Teleskopvorrichtung nahezu die gesamte Harnblase entleert werden kann, ohne dabei Rückstände in der Harnblase zu belassen.

Wird gemäß Anspruch 7 die Pumpe als eine Druckmittelpumpe ausgestaltet so wird der Vorteil erzielt, daß keine aufwendige Mechanik wie beispielsweise bei der Verwendung einer Teleskopvorrichtung in dem dritten Bereich integriert ist.

Wird gemäß Anspruch 8 die Pumpe als Schraubenpumpe ausgebildet, so wird ebenfalls der Vorteil erreicht, daß nahezu das gesamte Volumen des dritten Bereichs für die Harnblase verwendet werden kann. Zusätzlich ergibt sich bei der Verwendung einer Schraubenpumpe der Vorteil, daß evtl. kleinere Harnkristalle von der Schraubenpumpe zermahlen werden, so daß die zermahlenen Kristalle auch bei verengter Harnröhre ausgeschieden werden können.

Wird darüber hinaus gemäß Anspruch 9 eine Schraubenpumpe so angeordnet, daß sie evtl. seitlich zur Fluidröhre verschoben werden kann, wird der Vorteil erreicht, daß ein Zugang und eine Spülung des künstlichen Harnableitungssystems unproblematisch herbeigeführt werden kann, da durch das Wegbewegen einer Schraube die Fluidröhre freigegeben wird. Diese Möglichkeit des Zugangs und der Spülung des künstlichen Harnableitungssystems ist beispielsweise hinsichtlich spektroskopischer Untersuchungen von Bedeutung.

Wird gemäß Anspruch 10 ein Sphinktermechanismus vorzugsweise im ersten Bereich vorgesehen, so wird der Vorteil erreicht, daß eine nahezu vollständige Kontrolle der Kontinenz möglich ist. Die Kontrolle des Sphinktermechanismus kann beispielsweise auch extern ausgelöst werden.

Wird gemäß Anspruch 11 zusätzlich eine Steuerung vorgesehen, welche den Sphinktermechanismus regelt, so kann beispielsweise die Steuerung, welche zusätzlich weitere Funktionen übernehmen kann, auch das Öffnen und Schließen des Sphinkters regeln.

Wird gemäß Anspruch 12 eine Sensorik vorgesehen, welchen den Füllstand der Harnblase überwacht, so wird der betreffenden Person ein hoher Grad an Sicherheit im Umgang mit der künstlichen Harnblase gegeben. Das heißt, die Person muß nicht regelmäßig und in kurzen Abständen die Harnblase entleeren, sondern kann sich gewohnt in das Alltagsleben integrieren. Wird der betreffenden Person entweder ein akustisches oder seismisches Signal übermittelt, welches bei einer bestimmten Befüllung der Blase ausgelöst wird, so kann sich die Person normal im Alltagsleben bewegen. Allerdings sollte hierbei beachtet werden, daß zumindest eine Sicherungsregelung bei der Sensorik eingebaut ist, das heißt, sollte ein gewisser Zeitraum von beispielsweise 8 bis 12 Stunden überschritten werden, so sollte unabhängig von dem Befüllungsstand der Blase der Person signalisiert werden, eine Entleerung durchzuführen. Ferner kann somit eine an den physiologischen Randbedingungen orientierte Sicherheit bei Erfassung des Füllungszustands der Kunstblase gegeben werden. Dadurch wird erreicht, daß die künstliche Harnableitungseinrichtung ähnlich der Funktion der naturlichen Harnblase arbeitet. Das heißt, mit der anmeldungsgemäßen Harnableitungseinrichtung wird erreicht, daß ähnlich dem natürlichen Vorgang, der Körper der Person vorerst signalisiert, daß die Harnblase entleert werden sollte, dann die Harnblase geöffnet wird, der Harn ausgedrückt wird und dann wieder geschlossen wird.

Wird gemäß Anspruch 13 die Sensorik durch die für die natürliche Harnblase zuständigen Nerven gesteuert, so wird mit dieser neurologischen Lösung nahezu ein natürliches Empfinden der betroffenen Person vermittelt, d.h. ein körperfremdes Signal, wie es beispielsweise durch ein akkustisches oder saismisches Signal erzeugt wird, wird somit nicht erforderlich.

Wird gemäß Anspruch 14 zusätzlich eine Energieversorgung im Harnableitungssystem vorgesehen, so kann eine kompakte Harnableitungseinrichtung bereitgestellt werden, welche beispielsweise vorab in die künstliche Harnableitungseinrichtung integriert sein kann. Allerdings sei an dieser Stelle darauf hingewiesen, daß die Energieversorgung ebenfalls separat in der Nähe der Harnableitungseinrichtung im Patienten positioniert werden kann, sollte aus Platzgründen ein dritter Bereich verwendet werden müssen, welcher eine zusätzliche Energieversorgung nicht zuläßt.

Wird gemäß Anspruch 15 die Energieversorgung von einer externen Wiederaufladevorrichtung durchgeführt, so wird der Vorteil erreicht, daß nahezu lebenslang die Harnableitungseinrichtung mit Energie versorgt werden kann. Zum Aufladen der der externen Wiederaufladevorrichtung angepaßten Gegenstücks kann derart erfolgen, daß drahtlos transkutan an einer geeigneten Hautauflagestelle das Gegenstück lädt, welches subkutan implantiert ist.

Eine einfache Übertragung der Energie kann beispielsweise dadurch erreicht werden, daß die Wiederaufladevorrichtung induktiv mit dem Gegenstück zusammenarbeitet, wobei beispielsweise induktiv mit körperverträglichen Frequenzen, beispielsweise 30 kHz, Energie übertragen werden kann.

Wird gemäß Anspruch 17 die Energieversorgung durch in der Harnableitungseinrichtung integrierten Primärbatterien vorgenommen, so arbeitet die Harnableitungseinrichtung ohne weitergehende Wartung bzw. die Person muß sich über die Energieversorgung keine Gedanken machen.

An dieser Stelle sei ebenfalls darauf hingewiesen, daß beispielsweise die Energie für die Aktorik und/oder Sensorik im Bedarfsfall drahtlos transkutan durch Auflegen einer geeigneten Übertragungsvorrichtung auf die Haut übertragen werden kann. Hierbei ist es aber ebenfalls notwendig, daß als zusätzliche Energiequelle die Steuerung und Versorgung über Primärbatterien erfolgen kann. Auch ist hierbei denkbar, daß die gesamte Steuerung und Sensorik von außen telemetrisch abgefragt und in Gang gesetzt werden kann.

Wird gemäß Anspruch 18 zusätzlich eine Aktorik in die Harnableitungseinrichtung integriert, so wird erneut ein vollständig abgeschlossenes System bereitgestellt, welches lediglich an den Zugängen bzw. Ausgängen mit funktionellen Strukturen des Harnableitungssystems des Patienten verbunden werden muß und als kompaktes Teil eingesetzt werden kann.

Wird gemäß Anspruch 19 der dritte Bereich zweigeteilt aufgebaut, wobei der eine Teil sich entsprechend der Befüllung der Harnblase von dem anderen Teil wegbewegen kann, so kann beispielsweise je nach Anforderung die Harnblasengröße bzw. die Befüllung flexibel eingestellt werden.

Weist gemäß Anspruch 20 die Harnableitungseinrichtung zwei Zugänge im dritten Bereich auf, so daß jeder Harnleiter mit der künstlichen Harnleitereinrichtung verbunden werden kann, ist es diesbezüglich nicht notwendig, evtl. ein weiteres separates Zusatzelement beispielsweise in Y-Form vorzusehen, welches angewendet werden kann, wenn es von Vorteil ist, daß die Harnableitungseinrichtung lediglich einen Zugang hat.

Durch Vorsehen eines oder mehrerer Antireflux-Ventile im dritten Bereich, gemäß Anspruch 21, wird erreicht, daß ein Rückfließen des Harns in die Niere unterbunden wird. Insbesondere wird auch dadurch einem eventuellen Bakterienaufstieg aus der Blase in die Niere entgegengewirkt.

Wird gemäß Anspruch 22 ein Befestigungselement vorgesehen, so ist auf einfache Weise eine Lagepositionierung und -fixierung im menschlichen Körper möglich.

Wird gemäß Anspruch 23 das Befestigungselement über eine Schwalbenschwanzverbindung mit der Harnableitungseinrichtung verbunden, so ist eine verliersichere Verbindung hergestellt und das Befestigungselement kann vorab im Körper gehalten werden, um dann entsprechend an der richtigen Stelle mit der Harnableitungseinrichtung verbunden zu werden.

Wird gemäß Anspruch 24 das Befestigungselement über ein Führungssystem verschiebbar befestigt, so kann je nach Anatomie der betreffenden Person die Harnableitungseinrichtung optimal positioniert und fixiert werden. Wird darüber hinaus das Führungsschienensystem in den dritten Bereich integriert, so sind keine dem dritten Bereich vorstehenden Schienen vorhanden, welche ggf. die Positionierung bzw. Plazierung im menschlichen Körper beeinflussen würde oder eine funktionelle oder räumliche Beeinträchtigung herbeiführen würde.

Weist gemäß Anspruch 25 das Befestigungselement ein Spreizelement auf, welches beispielsweise in die Führungsschienen nach Einsetzen sich aufweitet, so ist eine einfache Verbindungsmöglichkeit gegeben, wobei insbesondere durch die vollständige Integrierung des Spreizelements in dem Befestigungselement eine gewisse Verträglichkeit gewährleistet wird.

Wird gemäß Anspruch 26 das Befestigungselement aus biokompatiblem Material, beispielsweise Silikon ausgebildet, so ist zum einen ein verträgliches Material vorgegeben und zum anderen wird aufgrund der Elastizität des Silikons etc. den Spreizbewegungen des Spreizelements Rechnung getragen.

Weitere vorteilhafte Ausgestaltung der vorliegenden Erfindung sind Gegenstand der übrigen Unteransprüche.

Unter Bezugnahme auf die nachfolgende Zeichnung wird das anmeldungsgemäße künstliche Harnableitungssystem detaillierter anhand einer bevorzugten Ausführungsform beschrieben.

Fig. 1    zeigt eine schematische Darstellung der anmeldungsgemäßen künstlichen Harnableitungseinrichtung;

Fig. 2    stellt eine Schnittzeichnung entlang der Schnittlinie II - II dar;

Fig. 3    zeigt eine Draufsicht des anmeldungsgemäßen Harnableitungssystems gemäß Fig. 1;

Fig. 4    zeigt eine Sicht von unten des anmeldungsgemäßen Harnableitungssystems;

Fig. 5    ist das anmeldungsgemäße Harnableitungssystem mit separierten einzelnen Bereichen gemäß Fig. 1;

Fig. 6    ist eine Schnittansicht der Positionierung des anmeldungsgemäßen Harnableitungssystems;

Fig. 7    ist eine Vorderansicht der anmeldungsgemäßen Harnableitungseinrichtung;

Fig.    8 ist eine Draufsicht eines Körperschnitts hinsichtlich der Schnittlinie VII - VII;

Fig.    9 zeigt ein Diagramm, welches die eine angeführte Polynomfunktion sechsten Grades hinsichtlich der Oberflächenkontur der Oberseite des anmeldungsgemäßen Harnableitungssystems gemäß Fig. 1 darstellt;

Fig.     10 zeigt ein Diagramm, welches die Silhouette bei Draufsicht des anmeldungsgemäßen Harnableitungssystems gemäß Fig. 1 im Verhältnis zu der angeführten Polynomfunktion sechsten Grades darstellt;

Fig.     11 zeigt eine Ausführungsform des Befestigungselements;

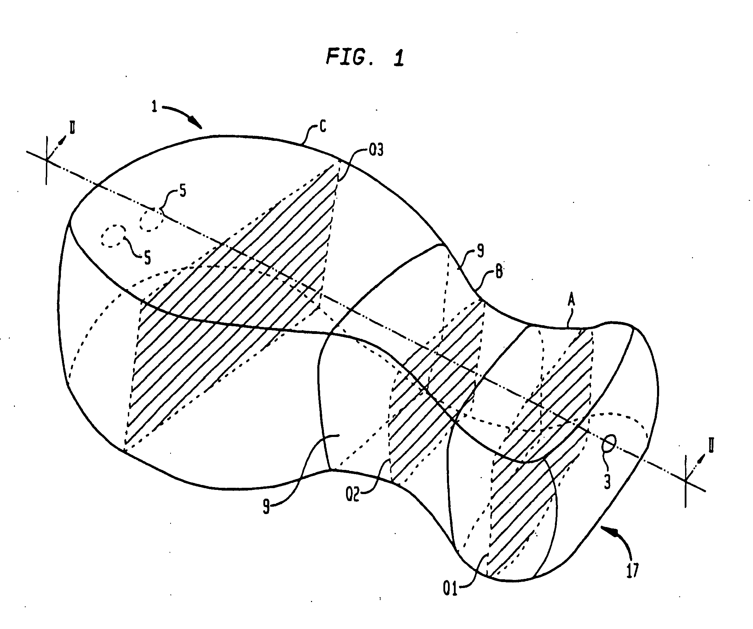

Die vorteilhafte Ausführungsform des in Fig. 1 dargestellten anmeldungsgemäßen Harnableitungssystems 1 weist einen ersten Bereich A, einen zweiten Bereich B und einen dritten Bereich C auf, wobei in dieser Ausführungsform die senkrecht zur Axialausrichtung bzw. Longitudinalrichtung der Harnableitungseinrichtung angeordneten Querschnittsflächen (schraffiert gezeichnet) des ersten, zweiten und dritten Bereiches so ausgestaltet sind, daß die maximale Querschnittsfläche Q1 des ersten Bereiches A größer als die minimale Querschnittsfläche Q2 des zweiten Bereiches B ist und die maximale Querschnittsfläche Q3 des dritten Bereiches C jeweils größer als die Querschnittsfläche des ersten und zweiten Bereiches ist. Zusätzlich zeigt der erste Bereich A einen Ausgang 3 sowie der dritte Bereich C zwei Zugänge 5 für die Harnröhren, welche von den jeweiligen Nieren kommen.

Der erste Bereich A der anmeldungsgemäßen Harnableitungseinrichtung 1 weist zu seiner Endfläche 7 einen sich erhöhenden Bereich D, wobei die Formgestaltung sowohl linear, bogenförmig, konkav oder konvex, je nachdem welche anatomischen Vorgaben der Patient an das Harnableitungssystem stellt, gestaltet sein kann. Deutlich ist in Fig. 1 zu sehen, daß der zweite Bereich B, welcher zwischen dem ersten und dritten Bereich angeordnet ist, als eine Einschnürung anzusehen ist, an deren seitlichen Flächen 9 die Arterien vorbeigeführt werden. Der dritte Bereich C, welcher eine Harnblase enthält, ist entsprechend voluminös ausgestaltet, um möglichst eine große Befüllung zuzulassen. An der Stirnseite des dritten Bereiches sind die beiden Zugänge für die Nierenharnröhren vorgesehen.

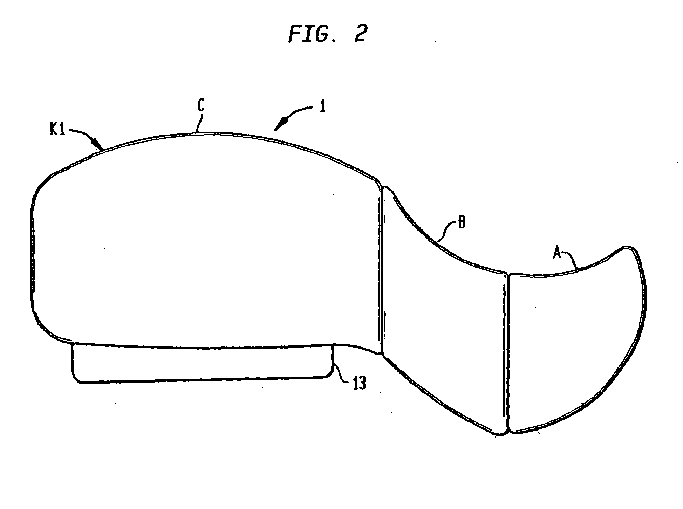

Fig. 2 zeigt eine seitliche Schnittansicht gemäß der Schnittlinie II - II. Anhand dieser Schnittansicht ist deutlich zu erkennen, daß die in Fig. 2 so präsentierte Harnableitungseinrichtung 1 die Oberseite eine erste Konturlinie K1 aufzeigt. Hierbei ist deutlicher als in Fig. 1 die Erhöhung von dem zweiten Bereich B zu der Endfläche 7 des ersten Bereiches A zu erkennen. In dieser Ausgestaltung ist eine kurvenförmige Erhöhung dargestellt. Diese kurvenförmige Erhöhung dient dazu, beispielsweise mit dem Schambein in Anlagekontakt gebraucht zu werden, und somit eine Lagefixierung zu ermöglichen. Ebenfalls ist in Fig. 2 zu erkennen, daß unterhalb des dritten Bereiches sogenannte Führungsschienen 13 vorgesehen sind, in die ein Befestigungselement (nicht gezeigt) einsetzbar ist. An dieser Stelle soll hervorgehoben werden, daß ein Hervorstehen der Führungsschienen beispielsweise durch vollständige Integration in den dritten Bereich vermieden werden kann.

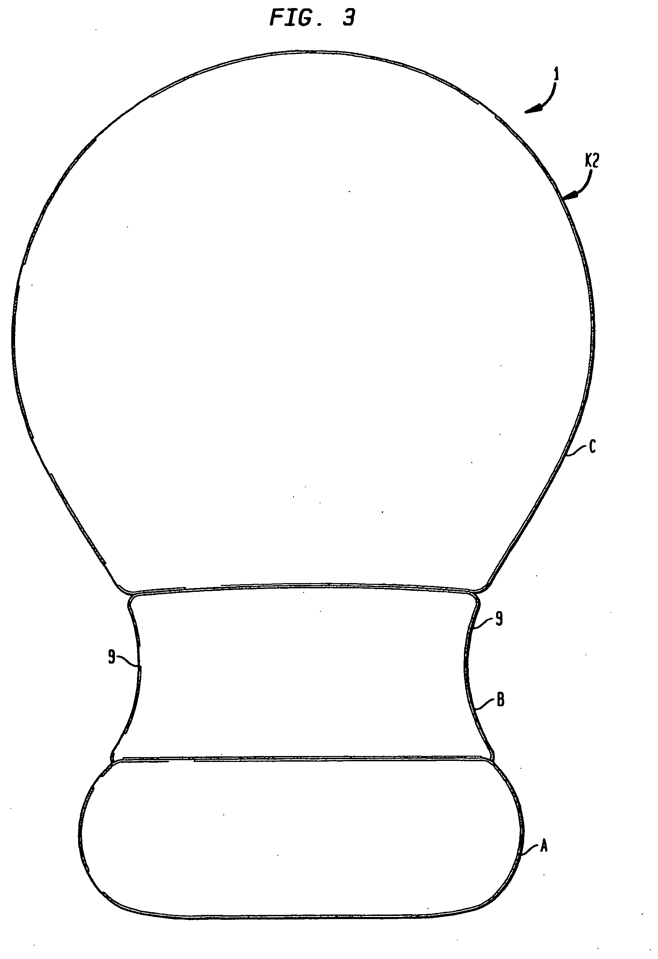

Fig. 3 zeigt eine Draufsicht des anmeldungsgemäßen Harnableitungssystems 1 und einer zweiten Konturlinie K2 gemäß Fig. 1, wobei deutlich die durch den zweiten Bereich B verursachte Einschnürung erkennbar ist, an der zu beiden Seiten entlang den seitlichen Flächen 9 die Arterien entlanggeführt werden können. Deutlich sind auch die Größenverhältnisse, die zwischen dem ersten, zweiten und dritten Bereich dargestellt sind, erkennbar.

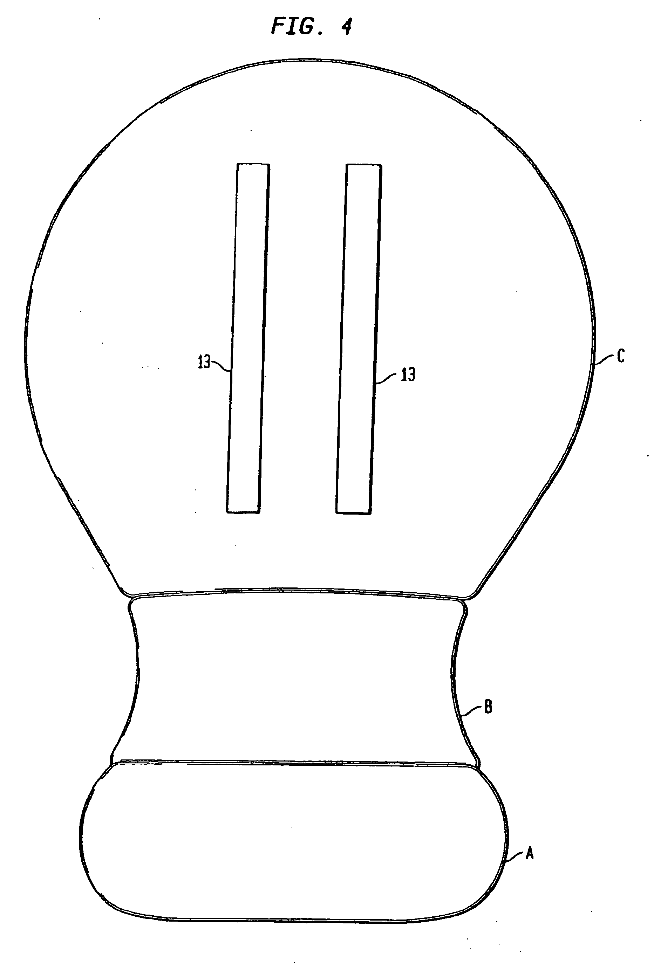

In Fig. 4 ist eine Sicht von unten hinsichtlich des anmeldungsgemäßen Harnableitungssystems 1 dargestellt. Deutlich sind die für das Befestigungselement vorgesehenen Führungsschienen 13 wiedergegeben.

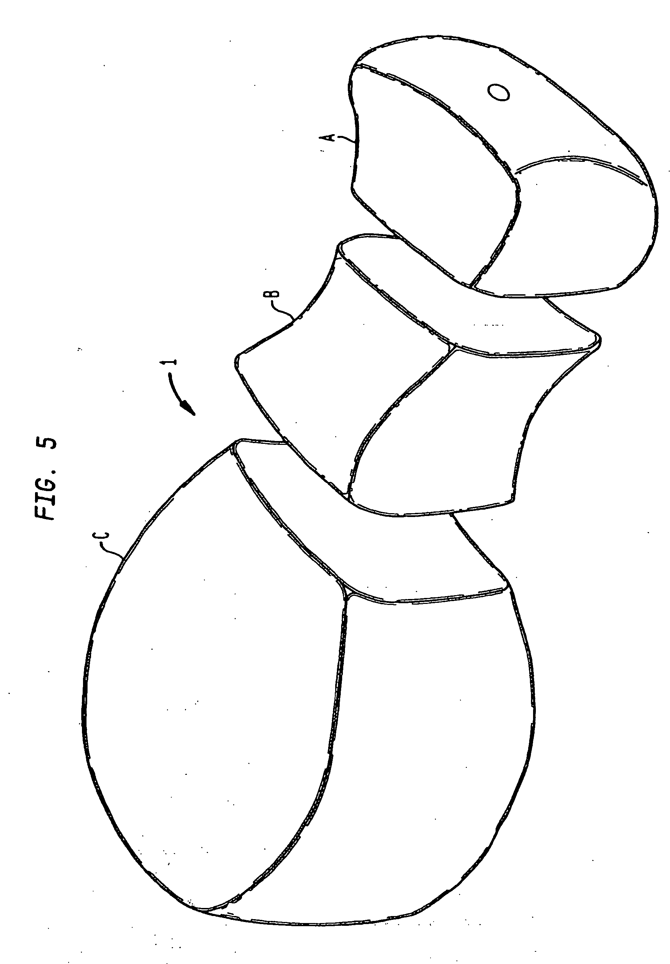

In Fig. 5 ist das anmeldungsgemäße Harnableitungssystem 1 mit seinen einzelnen Bereichen, d.h. erster, zweiter und dritter Bereich, separat dargestellt.

An dieser Stelle sei darauf hingewiesen, daß die Aufteilung in einen ersten Bereich, in einen zweiten Bereich und in einen dritten Bereich eine vorteilhafte Ausführungsform darstellt. Ferner sind auch mehr als drei Bereiche, welche separat getrennt werden können, vorstellbar, wodurch mehreren Bereichen der erhöhten Anpassungsvariation Rechnung getragen wird.

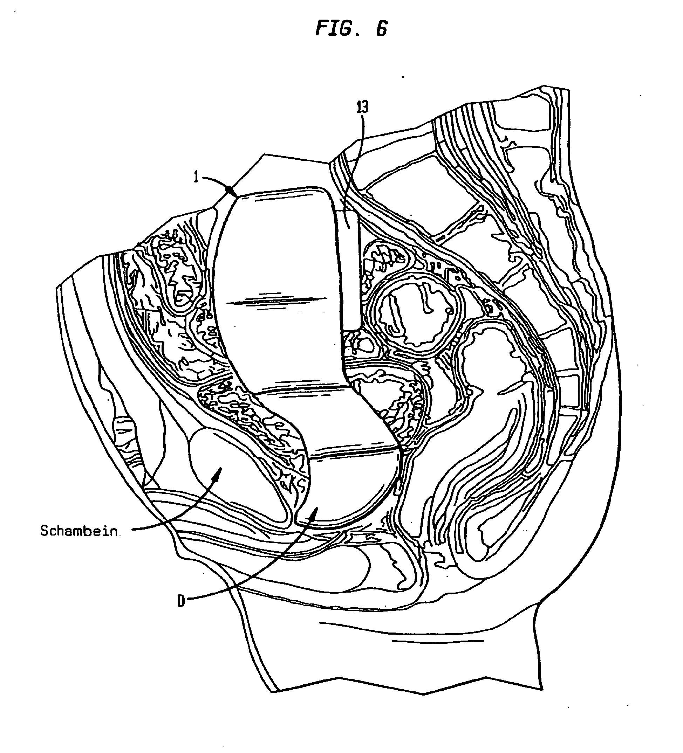

In Fig. 6 ist beispielsweise die Lagepositionierung des anmeldungsgemäßen, Harnableitungssystems dargestellt. Der erste Bereich A liegt am Schambein an, wobei das Befestigungselement, welches verschiebbar in den Führungsschienen aufnehmbar ist, beispielsweise an entsprechenden Stellen in der Bauchhöhle befestigt werden.

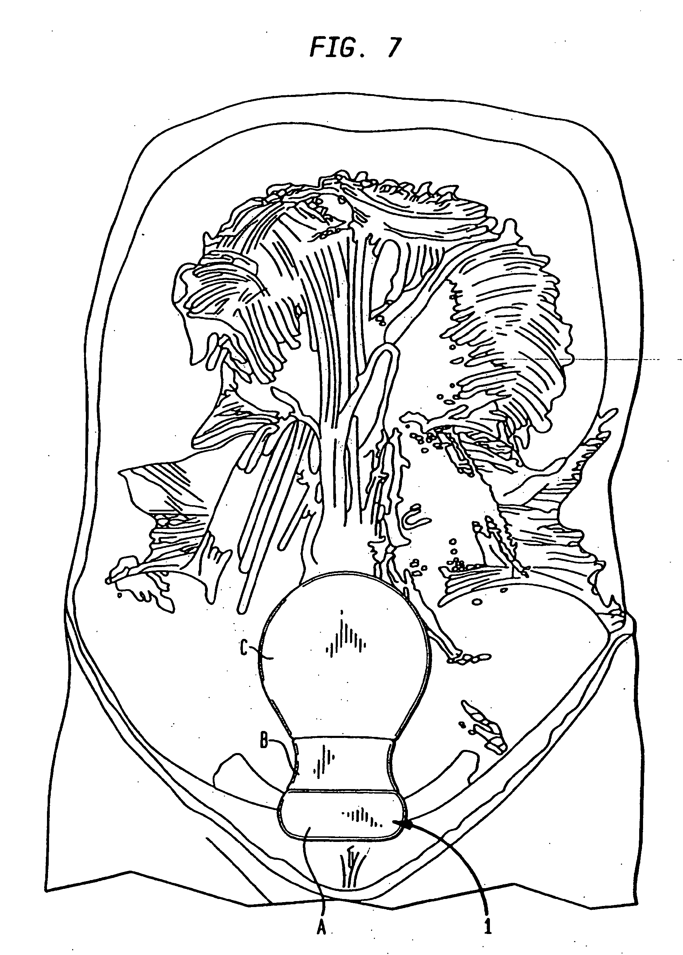

Zur weiteren Veranschaulichung ist in Fig. 7 eine Vorderansicht gezeigt, wie die Lagepositionierung des anmeldungsgemäßen Harnableitungssystems möglich ist.

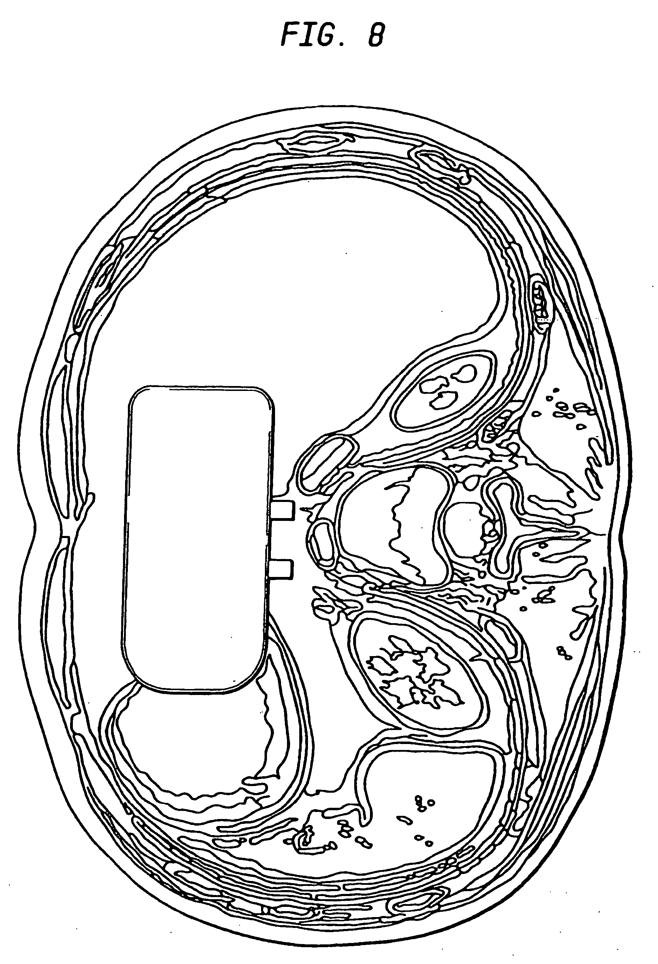

Fig. 8 zeigt eine Draufsicht, wobei der Körperschnitt oberhalb des Schnitts des anmeldungsgemäßen Harn- bleitungssystems liegt.

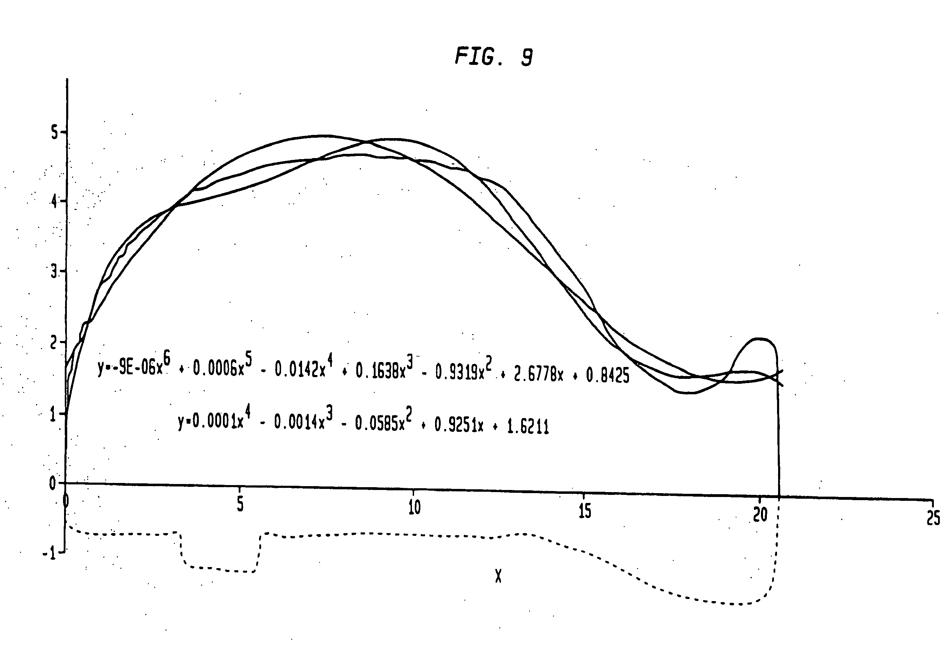

In Fig. 9 ist beispielsweise eine Fitkurve der Polynomform $f(x) = a_6 x^6 + a_5 x^5 + a_4 x^4 + a_3 x^3 + a_2 x^2 + a_1 x + a$ dargestellt, d.h. ein Polynom sechsten Grades, welches an die erste Konturlinie angepaßt wurde. Die für diese An- passung verwendeten Parameter sind $a_6 = -9 \cdot 10^6$; $a_5 = 0{,}006$; $a_4 = -0{,}014$; $a_3 = 0, 1638$; $a_2 = -0{,}9319$, $a_1 = 2{,}6778$ und $a = 0{,}8425$. Allerdings hat sich gezeigt, daß innerhalb eines Definitionsbereiches von $0 \leq x \leq 22$ die Koeffizienten $a_1$ bis $a_6$ in den Bereichen $0<A<2$; $0<a_1<8$; $-2<a_2<0$; $0<a_3<1$; $-0, 1<a_4<0$; $0<a_5<0, 003$; und $-0.00001<a_6<0$ innerhalb eines Definitionsbereichs von $0<x<22$ genommen werden können.

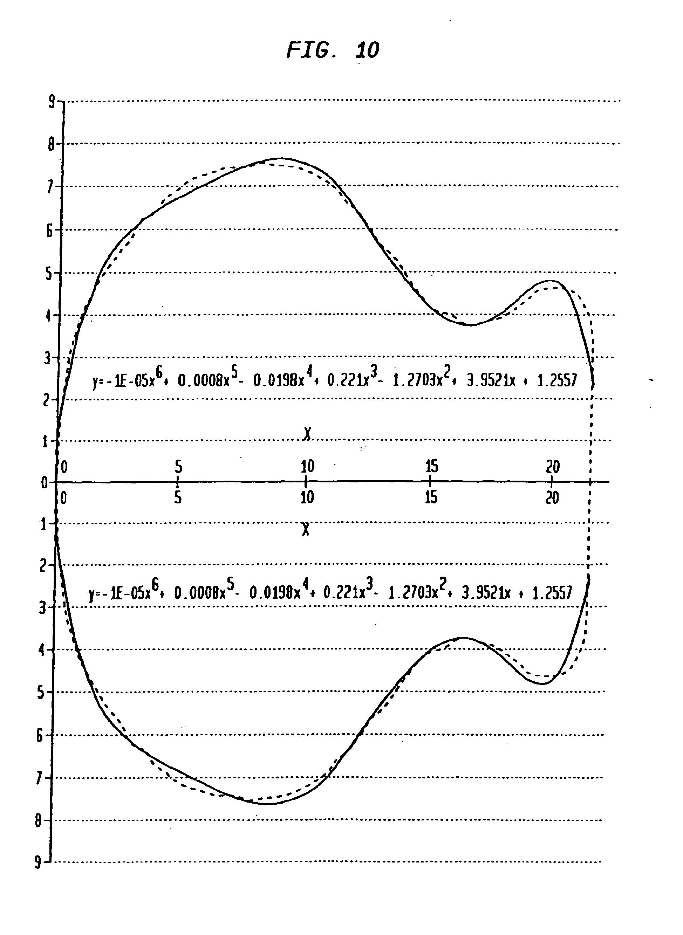

In Fig. 10 ist eine hälftige zweite Konturlinie bei Draufsicht dargestellt, welche ebenfalls mit einem Polynom sechsten Grades angenähert wurde. Die hierfür verwendeten Parameter waren $a_6 = 1 \cdot 10^{-5}$ ; $a_5 = 0{,}008$; $a_4 = -0{,}0198$; $a_3 = 0{,}221$; $a_2 = -1{,}2703$; $a_1 = 3{,}9521$ und $A = 1{,}2557$. Es hat sich ebenso gezeigt, daß diese Koeffizienten ebenfalls in den Bereichen $0<A<2$; $0<a_1<8$ ; $-2<a_2<0$; $0<a_3<1$; $-0{,}1<a_4<0$; $0<a_5<0{,}003$; und $-0.00001<a_6<0$ innerhalb eines Defi-

nitionsbereichs von 0<x<22 genommen werden können, um die entsprechende zweite Konturlinie anzupassen. Zur Verdeutlichung, daß Fig. 10 eine Draufsicht ist, wurde die erste Konturlinie und die gefittete Kurve bei y = 0 an der x-Achse des Diagramms gespiegelt.

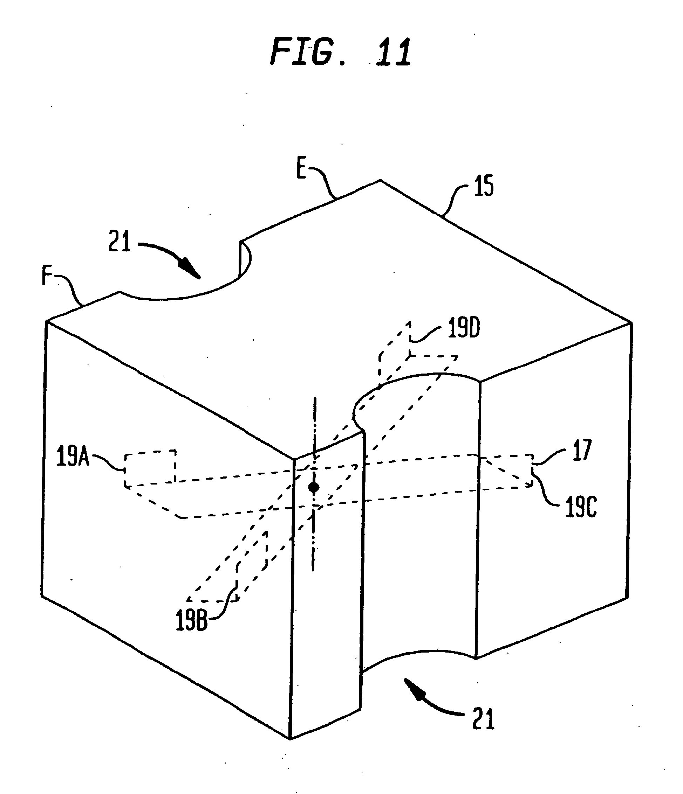

In Fig. 11 ist ein Befestigungselement 15 dargestellt, welches einen vorderen Bereich F hat, welcher in die Führungsschienen des anmeldungsgemäßen Harnableitungssystems eingeführt werden können, und einen Endbereich E hat, welcher beispielsweise mit der Hand zusammengedrückt werden kann.

Innerhalb dieses Befestigungselements 15 ist ein Spreizelement 17 (gestrichelt dargestellt), welches aufgrund der hochgestellten Seitenflächen 19A bis 19D von dem beispielsweise elastisch ausgebildeten Befestigungselement 15 mitgenommen wird, so daß beispielsweise beim Zusammendrücken des Endbereiches die Schenkel des Spreizelements 17 im vorderen Bereich ebenfalls aufeinander zu bewegt werden und das elastische Material des Befestigungselementes 15 mitnehmen.

Auf diese Weise kann das Befestigungselement 15 soweit verengt werden, daß es zwischen die beiden Führungsschienen 13 einbringbar ist. Nach Einführung wird dann das Befestigungselement 15 freigegeben, so daß aufgrund der Elastizität des Befestigungselementes 15 der vordere Bereich F wieder in seine Ursprungsform zurückgeführt wird, und eine Preßpassung mit den Seitenwänden der Führungsschienen 13 erzielbar ist. Sollte nun das Befestigungselement 15 innerhalb der Führungsschiene 13 verschoben werden, so muß lediglich erneut der Endbereich E zusammengedrückt werden, um somit die Preßpassung der seitlichen Flächen des vorderen Bereiches F zu lösen. Die Ausnehmungen 21 in dem Befestigungselement 13 dienen dazu, von den Führungsschienen verlierungssicher geführt zu werden, wenn die Position des Befestigungselements gegebenenfalls nachjustiert wird.

Mit diesem Befestigungselement 15 kann somit die anmeldungsgemäße Harnableitungseinrichtung vor seiner endgültigen Lagefixierung entsprechend ausgerichtet werden und das Befestigungselement in der Bauchhöhle an der entsprechenden Position fixiert werden.

Aufgrund dieser zusätzlichen Maßnahme, ein Befestigungselement separat zur Harnableitungseinrichtung bereitzustellen, kann beispielsweise auch bei nicht einfach zugänglichen Stellen für die Befestigung des Befestigungselementes, das Befestigungselement vorab fixiert werden und dann die Harnableitungseinrichtung eingeführt werden.

Anstelle der Schraubenpumpe, der eine Teleskopvorrichtung verwendenden Pumpe und der Druckmittelpumpe sind alle weiteren Formen von Pumpen für das Anpressen der Harnflüssigkeit denkbar, insbesondere aber auch eine Membranpumpe bzw. Zahnradpumpe.

Die Querschnittsflächen Q1, Q2 und Q3 können unterschiedliche geometrische Flächen aufweisen, beispielsweise quadratisch, rechteckig, trapezförmig, kreisförmig, oval, elliptisch oder eine Kombination davon.

## Patentansprüche

1. Künstliche Harnableitungseinrichtung mit Longitudinalrichtung aus zumindest einem ersten Bereich mit maximaler Querschnittsfläche $Q_1$ mit zumindest einem Ausgang einem zweiten Bereich mit minimaler Querschnittsfläche $Q_2$ und einem dritten Bereich mit maximaler Querschnittsfläche $Q_3$ und mit zumindest einem zugang zur Aufnahme einer Harnblase und welche in dem dritten Bereich eine Harnblase aufweist. wobei der zweite Bereich (B) zwischen dem ersten und dem dritten Bereich (A, C) und der erste, zweite und dritte Bereich entlang der Longitudinalrichtung angeordnet sind ,wobei die senkrecht zur Longitudinalrichtung der Harnableitungseinrichtung angeordneten Querschnittsfläche Q1, Q2 des ersten und/oder zweiten Bereichs (A, B) kleiner als die Querschnittsfläche Q3 des dritten Bereichs (C) ist,
   wobei die Querschnittsfläche Q1 des ersten Bereichs (A) größer als die Querschnittsfläche Q2 des zweiten Bereichs (B) ist.

2. Harnableitungseinrichtung nach Anspruch 1, **dadurch gekennzeichnet, dass** der erste, der zweite und dritte Bereich (A, B, C) modular zusammengesetzt werden kann, wobei jede Übergangsfläche eine Grundfläche definiert, die einen stetigen Übergang der Oberflächen der Harnableitungseinrichtung erlaubt.

3. Harnableitungseinrichtung nach Anspruch 1 oder 2, **dadurch gekennzeichnet, dass** ein Aktor und ein Sphinktermechanismus in der Harnableitungseinrichtung vorgesehen ist.

4. Harnableitungseinrichtung nach einem der Ansprüche 1 bis 3, **dadurch gekennzeichnet, dass** eine Fluidführung vorgesehen ist, welche sich von der Harnblase vorzugsweise von dem dritten Bereich (C) über den zweiten Bereich (B) durch den ersten Bereich (A) erstreckt.

5. Harnableitungseinrichtung nach einem der Ansprüche 2 bis 4, **dadurch gekennzeichnet, dass** der Aktor eine Pumpe ist, und vorzugsweise im dritten Bereich vorgesehen ist.

6. Harnableitungseinrichtung nach Anspruch 5, **dadurch gekennzeichnet, dass** die Pumpe eine Teleskopvorrichtung ist.

7. Harnableitungseinrichtung nach Anspruch 5, **dadurch gekennzeichnet, dass** die Pumpe eine aus zwei Kammern aufgebaute Druckmittelpumpe ist.

8. Harnableitungseinrichtung nach Anspruch 5, **dadurch gekennzeichnet, dass** die Pumpe eine Schraubenpumpe ist.

9. Harnableitungseinrichtung nach Anspruch 8, **dadurch gekennzeichnet, dass** die eine Schraube der Schraubenpumpe sich seitlich verschieben lässt.

10. Harnableitungseinrichtung nach einem der Ansprüche 1 bis 9, **dadurch gekennzeichnet, dass** eine Steuerung vorgesehen ist, welche vorzugsweise einen Sphinktermechanismus regelt.

11. Harnableitungseinrichtung nach einem der Ansprüche 1 bis 10, **dadurch gekennzeichnet, dass** eine Sensorik vorgesehen ist, welche den Füllstand der Harnblase überwacht und vorzugsweise ein akustisches bzw. seismisches Signal bei Erreichen einer bestimmten Befüllung der Blase auslöst.

12. Harnableitungseinrichtung nach Anspruch 11, **dadurch gekennzeichnet, dass** die Sensorik durch die für die natürliche Harnblase zuständigen Nerven gesteuert wird.

13. Harnableitungseinrichtung nach einem der Ansprüche 1 bis 12, **dadurch gekennzeichnet, dass** eine Energieversorgung vorgesehen ist.

14. Harnableitungseinrichtung nach Anspruch 14, **dadurch gekennzeichnet, dass** die Energieversorgung durch eine externe Wiederaufladevorrichtung erfolgt, welche mit einem Gegenstück, welches mit der Harnableitungseinrichtung in Verbindung steht, zusammenarbeitet.

15. Harnableitungseinrichtung nach Anspruch 14, **dadurch gekennzeichnet, dass** die Wiederaufladevorrichtung induktiv mit dem Gegenstück zusammenarbeitet.

16. Harnableitungseinrichtung nach Anspruch 13, **dadurch gekennzeichnet, dass** die Energieversorgung durch in der Harnableitungseinrichtung integrierte Primärbatterien erfolgt.

17. Harnableitungseinrichtung nach einem der Ansprüche 1 bis 16, **dadurch gekennzeichnet, dass** eine Aktorik vorgesehen ist, welche das Auspressen des Harns durchführt.

18. Harnableitungseinrichtung nach einem der Ansprüche 1 bis 17, **dadurch gekennzeichnet, dass** ein fluidisches Teil im dritten Bereich vorgesehen ist, das zweigeteilt ist, wobei der eine Teil sich entsprechend der Befüllung von dem anderen Teil wegbewegen kann.

19. Harnableitungseinrichtung nach einem der Ansprüche 1 bis 18, **dadurch gekennzeichnet, dass** der dritte Bereich einen oder zwei Zugänge aufweist.

20. Harnableitungseinrichtung nach einem der Ansprüche 1 bis 19, **dadurch gekennzeichnet, dass** ein oder zwei Antireflux-Ventile vorgesehen sind, welche vorzugsweise in dem dritten Bereich angeordnet sind.

21. Harnableitungseinrichtung nach einem der Ansprüche 1 bis 20, **dadurch gekennzeichnet, dass** ein Befestigungselement vorgesehen ist.

22. Harnableitungseinrichtung nach Anspruch 21, **dadurch gekennzeichnet, dass** das Befestigungselement über eine Schwalbenschwanzverbindung mit der Harnableitungseinrichtung verbunden ist.

23. Harnableitungseinrichtung nach einem der Ansprüche 21 oder 22, **gekennzeichnet durch** ein Führungsschienensystem, in das das Befestigungselement verschiebbar aufgenommen werden kann und an einer geeigneten Stelle arretiert werden kann, wobei das Führungsschienensystem vorzugsweise in den dritten Bereich integriert ist.

24. Harnableitungseinrichtung nach einem der Ansprüche 21 bis 23, **dadurch gekennzeichnet, dass** das Befestigungselement ein Spreizelement enthält, welches vorzugsweise vollständig eingeschlossen ist.

25. Harnableitungseinrichtung nach Anspruch 24, **dadurch gekennzeichnet, dass** das Befestigungselement aus biokompatiblem, elastischem Material, vorzugsweise aus Silikon gebildet ist.

26. Harnableitungseinrichtung nach einem der Ansprüche 1 bis 25, **dadurch gekennzeichnet, dass** entlang einer ersten Konturlinie die Form der Harnableitungseinrichtung einer Polynom-Funktion sechsten Grades entspricht,

$$F(x) = A + a_1x + a_2x^2 + a_3x^3 + a_4x^4 + a_5x^5 + a_6x^6$$

mit den Koeffizienten in den Bereichen $0<A<2$; $0<a_1<8$; $-2<a_2<0$; $0<a_3<1$; $-0,1<a_4<0$; $0<a_5<0,003$; und $-0,00001<a_6<0$ innerhalb eines Definitionsbereichs von $0<x<22$.

27. Harnableitungseinrichtung nach einem der Ansprüche 1 bis 26, **dadurch gekennzeichnet, dass** entlang einer zweiten Konturlinie die Form der Harnableitungseinrichtung einer Polynom-Funktion sechsten Grades entspricht,

$$F(x) = A + a_1x + a_2x^2 + a_3x^3 + a_4x^4 + a_5x^5 + a_6x^6$$

mit den Koeffizienten in den Bereichen $0<A<2$; $0<a_1<8$; $-2<a_2<0$; $0<a_3<1$; $-0,1 <a_4<0$; $0<a_5<0,003$; und $-0,00001<a_6<0$ innerhalb eines Definitionsbereichs von $0<x<22$.

28. Harnableitungseinrichtung nach einem der Ansprüche 1 bis 27, **dadurch gekennzeichnet, dass** der erste, der zweite und der dritte Bereich integral ausgebildet sind.

**Claims**

1. Artificial urinary diversion device with longitudinal alignment consisting of at least one first area with a maximum cross-sectional surface Q1 with at least one outlet, one second area with a minimum cross-sectional surface Q2 and one third area with a maximum cross-sectional surface Q3 and with at least one inlet for acceptance of a urinary bladder, and which shows in the third area a urinary bladder, wherein the second area (B) is arranged between the first and the third area (A,C) and the first, second and third area are arranged along the longitudinal alignment, wherein the cross-sectional surfaces (Q1,Q2) of the first and/or second area (A,B) perpendicular to the longitudinal alignment of the urinary diversion device are smaller than the cross-sectional surface (Q3) of the third area (C), wherein the cross-sectional surface (Q1) of the first area (A) being larger than the cross-sectional surface (Q2) of the second area (B).

2. Urinary diversion device according to claim 1, **characterized in that** the first, the second and the third area (A,B, C) can be compounded modularly, with each transition area defining a basic area, permitting a continuous transition of the surfaces of the urinary diversion system.

3. Urinary diversion device according to claim 1 or 2, **characterized in that** an actor and a sphincter mechanism is provided in the urinary diversion device.

4. Urinary diversion system according to one of the claims 1 to 3, **characterized in that** a fluid-guidance is provided which extends from the urinary bladder preferably from the third area (C) to the second area (B) via the first area (A).

5. Urinary diversion device according to one of the claims 2 to 4, **characterized in that** the actor is a pump, which is preferably arranged in the third area.

6. Urinary diversion device according to claim 5, **characterized in that** the pump is a telescope device.

7. Urinary diversion device according to claim 5, **characterized in that** the pump is a pump with pressurizing medium constructed with two chambers.

8. Urinary diversion device according to claim 5, **characterized in that** the pump is a screw pump.

9. Urinary diversion device according to claim 8, **characterized in that** one of the screws of the screw pump can be moved laterally.

10. Urinary diversion device according to one of the claims 1 to 9, **characterized in that** a control is provided, which preferably controls a sphincter mechanism.

11. Urinary diversion device according to one of the claims 1 to 10, **characterized in that** a sensor system is provided, which monitors the filling level of the urinary bladder and preferably produces a sound signal or a seismical signal when reaching a certain filling level of the bladder.

12. Urinary diversion device according to claim 11 **characterized in that** the sensor system is controlled by the nerves responsible for the natural urinary bladder.

13. Urinary diversion device according to one of the claims 1 to 12, **characterized in that** a power supply is provided.

14. Urinary diversion device according to claim 13, **characterized in that** an external recharge device, which cooperates with a counterpart that is connected with the urinary diversion device, makes the power supply.

15. Urinary diversion device according to claim 14, **characterized in that** the recharge device cooperates inductively with the counterpart.

16. Urinary diversion device according to claim 13 **characterized in that** the power supply is made by primary batteries that are integrated into the urinary diversion device.

17. Urinary diversion device according to one of the claims 1 to 16, **characterized in that** an actor system is provided, which executes the pressing out of the urine.

18. Urinary diversion device according to one of the claims 1 to 17, **characterized in that** a two-part fluidic part is provided in the third area wherein, depending on the filling level, one part is able to move away from the other part.

19. Urinary diversion device according to one of the claims 1 to 18, **characterized in that** the third area shows one or two inlets.

20. Urinary diversion device according to one of the claims 1 to 19, **characterized in that** one or two anti-reflux valves are provided, which are preferably arranged in the third area.

21. Urinary diversion device according to one of the claims 1 to 20, **characterized in that** a fixing element is provided.

22. Urinary diversion device according to claim 21, **characterized in that** the fixing element is connected with the urinary diversion device via a dovetail joint.

23. Urinary diversion device according to claim 21 or 22, **characterized by** a guide-rail system, in which the fixing element can be moveably included and locked at a suitable position, and the guide-rail system being preferably integrated into the third area.

24. Urinary diversion device according to one of the claims 21 to 23, **characterized in that** the fixing element comprises a splay or expanding element, which is preferably totally included.

25. Urinary diversion device according to claim 24, **characterized in that** the fixing element is made of biocompatible, elastic material, preferably silicone.

26. Urinary diversion device according to one of the claims 1 to 25, **characterized in that**, among a first outline, the shape of the urinary diversion device corresponds to a polynomial function of $6^{th}$ degree

$$F(x) = A + a_1x + a_2x^2 + a_3x^3 + a_4x^4 + a_5x^5 + a_6x^6$$

with the coefficients in the domains $0<A<2$; $0<a_1<8$; $-2<a_2<0$; $0<a_3<1$; $-0,1<a_4<0$; $0<a_5<0,003$; and $-0,00001<a_6<0$ within a domain of $0<x<22$.

27. Urinary diversion device according to one of the claims 1 to 26, **characterized in that**, among a second outline, the shape of the urinary diversion device corresponds to a polynomial function of $6^{th}$ degree,

$$F(x) = A + a_1x + a_2x^2 + a_3x^3 + a_4x^4 + a_5x^5 + a_6x^6$$

with the coefficients in the domains $0<A<2$; $0<a_1<8$; $-2<a_2<0$; $0<a_3<1$; $-0,1<a_4<0$; $0<a_5<0,003$; and $-0,00001 <a_6<0$ within a domain of $0<x<22$.

28. Urinary diversion device according to one of the claims 1 to 27, **characterized in that** the first, the second and the third area are formed integrally.

**Revendications**

1. Dispositif artificiel de dérivation urinaire à direction longitudinale, comprenant au moins une première zone à superficie de section transversale maximale Q1 pourvue d'au moins une sortie, une deuxième zone à superficie de section transversale minimale Q2 et une troisième zone à superficie de section transversale maximale Q3 pourvue d'au moins une entrée destinée à recevoir une vessie, lequel dispositif présente une vessie dans la troisième zone, la deuxième zone (B) étant située entre la première et la troisième zone (A, C) et la première, la deuxième et la troisième zone étant situées le long de la direction longitudinale, la superficie de section transversale Q1, Q2 de la première et/ou de la deuxième zone (A, B) qui est perpendiculaire à la direction longitudinale du dispositif de dérivation urinaire est plus petite que la superficie de section transversale Q3 de la troisième zone (C), la superficie de section transversale Q1 de la première zone (A) étant plus grande que la superficie de section transversale Q2 de la deuxième zone (B).

2. Dispositif de dérivation urinaire selon la revendication 1, **caractérisé en ce que** la première, la deuxième et la troisième zone (A, B, C) peuvent être assemblées de manière modulaire, chaque zone de jonction définissant une surface de base permettant une jonction continuelle entre les surfaces du dispositif de dérivation urinaire.

3. Dispositif de dérivation urinaire selon l'une des revendications 1 ou 2, **caractérisé en ce que** le dispositif de dérivation urinaire présente un actionneur et un mécanisme de sphincter.

4. Dispositif de dérivation urinaire selon l'une des revendications 1 à 3, **caractérisé en ce qu'**il est prévu une conduite de fluide partant de la vessie, de préférence de la troisième zone (C) en passant par la deuxième zone (B) pour traverser la première zone (A).

5. Dispositif de dérivation urinaire selon l'une des revendications 2 à 4, **caractérisé en ce que** l'actionneur est une pompe prévue de préférence dans la troisième zone.

6. Dispositif de dérivation urinaire selon la revendication 5, **caractérisé en ce que** la pompe est un dispositif télescopique.

7. Dispositif de dérivation urinaire selon la revendication 5, **caractérisé en ce que** la pompe est une pompe hydraulique composée de deux chambres.

8. Dispositif de dérivation urinaire selon la revendication 5, **caractérisé en ce que** la pompe est une pompe à vis.

9. Dispositif de dérivation urinaire selon la revendication 8, **caractérisé en ce qu'**une vis de la pompe à vis peut se déplacer latéralement.

**10.** Dispositif de dérivation urinaire selon l'une des revendications 1 à 9, **caractérisé en ce qu'**il est prévu un organe de commande contrôlant de préférence un mécanisme de sphincter.

**11.** Dispositif de dérivation urinaire selon l'une des revendications 1 à 10, **caractérisé en ce qu'**il est prévu un système de capteur qui surveille le niveau de remplissage de la vessie et déclenche de préférence un signal acoustique ou sismique lorsque la vessie a atteint un certain niveau de remplissage.

**12.** Dispositif de dérivation urinaire selon la revendication 11, **caractérisé en ce que** le système de capteur est commandé par les nerfs existants pour la vessie naturelle.

**13.** Dispositif de dérivation urinaire selon l'une des revendications 1 à 12, **caractérisé en ce qu'**il est prévu une alimentation en énergie.

**14.** Dispositif de dérivation urinaire selon la revendication 13, **caractérisé en ce que** l'alimentation en énergie s'effectue au moyen d'un dispositif rechargeable externe coopérant avec un élément complémentaire relié au dispositif de dérivation urinaire.

**15.** Dispositif de dérivation urinaire selon la revendication 14, **caractérisé en ce que** le dispositif rechargeable externe coopère de manière inductive avec l'élément complémentaire.

**16.** Dispositif de dérivation urinaire selon la revendication 13, **caractérisé en ce que** l'alimentation en énergie s'effectue au moyen de piles primaires intégrées dans le dispositif de dérivation urinaire.

**17.** Dispositif de dérivation urinaire selon l'une des revendications 1 à 16, **caractérisé en ce qu'**il est prévu un système d'actionneurs effectuant l'évacuation de l'urine.

**18.** Dispositif de dérivation urinaire selon l'une des revendications 1 à 17, **caractérisé en ce qu'**une partie fluidique est divisée en deux parties dans la troisième zone, l'une de ces parties pouvant se déplacer par rapport à l'autre partie en fonction du remplissage de la vessie.

**19.** Dispositif de dérivation urinaire selon l'une des revendications 1 à 18, **caractérisé en ce que** la troisième zone présente une ou deux entrées.

**20.** Dispositif de dérivation urinaire selon l'une des revendications 1 à 19, **caractérisé en ce qu'**il est prévu un ou deux clapets anti-reflux situés de préférence dans la troisième zone.

**21.** Dispositif de dérivation urinaire selon l'une des revendications 1 à 20, **caractérisé en ce qu'**il est prévu un dispositif de fixation.

**22.** Dispositif de dérivation urinaire selon la revendication 21, **caractérisé en ce que** le dispositif de fixation est lié au dispositif de dérivation urinaire au moyen d'un assemblage à queue d'aronde.

**23.** Dispositif de dérivation urinaire selon l'une des revendications 21 ou 22, **caractérisé par** un système de rails de guidage dans lequel est admis le dispositif de fixation pour pouvoir y coulisser et être arrêté en une position appropriée, le système de rails de guidage étant de préférence intégré dans la troisième zone.

**24.** Dispositif de dérivation urinaire selon l'une des revendications 21 à 23, **caractérisé en ce que** le dispositif de fixation comprend un élément extensible qui est de préférence entièrement enclos.

**25.** Dispositif de dérivation urinaire selon la revendication 24, **caractérisé en ce que** le dispositif de fixation se compose de matériau biocompatible élastique, de préférence de silicone.

**26.** Dispositif de dérivation urinaire selon l'une des revendications 1 à 25, **caractérisé en ce que** la forme du dispositif de dérivation urinaire correspond, le long d'une première ligne de contour, à une fonction polynôme du sixième degré,

$$F(x) = A + a_1x + a_2x^2 + a_3x^3 + a_4x^4 + a_5x^5 + a_6x^6$$

avec des coefficients compris dans les plages $0 < A < 2$ ; $0 < a_1 < 8$ ; $-2 < a_2 < 0$ ; $0 < a_3 < 1$ ; $-0,1 < a_4 < 0$ ; $0 < a_5 < 0,003$ ; et $0,00001 < a_6 < 0$ dans une plage de définition de $0 < x < 22$.

27. Dispositif de dérivation urinaire selon l'une des revendications 1 à 26, **caractérisé en ce que** la forme du dispositif de dérivation urinaire correspond, le long d'une deuxième ligne de contour, à une fonction polynôme du sixième degré,

$$F(x) = A + a_1x + a_2x^2 + a_3x^3 + a_4x^4 + a_5x^5 + a_6x^6$$

avec des coefficients compris dans les plages $0<A<2$ ; $0<a_1<8$ ; $-2<a_2<0$ ; $0 < a_3 < 1$ ; $-0,1 <a_4 < 0$ ; $0 <a_5 < 0,003$ ; et $0,00001 < a_6 < 0$ dans une plage de définition de $0 < x < 22$.

28. Dispositif de dérivation urinaire selon l'une des revendications 1 à 27, **caractérisé en ce que** la première, la deuxième et la troisième zone sont conçues d'une seule pièce.

FIG. 1

EP 1 161 202 B1

FIG. 2

# FIG. 3

FIG. 4

FIG. 5

## FIG. 6

Schambein

## FIG. 7

## FIG. 8

FIG. 9

$y = -9E-06x^6 + 0.0006x^5 - 0.0142x^4 + 0.1638x^3 - 0.9319x^2 + 2.6778x + 0.8425$

$y = 0.0001x^4 - 0.0014x^3 - 0.0585x^2 + 0.9251x + 1.6211$

X

EP 1 161 202 B1

## FIG. 10

$$y = -1E-05x^6 + 0.0008x^5 - 0.0198x^4 + 0.221x^3 - 1.2703x^2 + 3.9521x + 1.2557$$

$$y = -1E-05x^6 + 0.0008x^5 - 0.0198x^4 + 0.221x^3 - 1.2703x^2 + 3.9521x + 1.2557$$

# FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5813410 A
- WO 9835633 A